# EUROPEAN PATENT APPLICATION

(11) **EP 2 295 034 A2**
(43) Date of publication of application: **16.03.2011**
(21) Application number: 10250443.8
(22) Date of filing: 10.03.2010
(51) Int. Cl.: A61K 8/89, A61K 8/898, A61Q 5/12

(54) **Cosmetic hair preparation**

(30) Priority: 25.03.2009 JP 2009073202
(71) Applicant: Shin-Etsu Chemical Co., Ltd., Chiyoda-ku, Tokyo (JP)
(72) Inventor: Hirai, Motohiko, Annaka-shi, Gunma-ken (JP)
(74) Representative: Stoner, Gerard Patrick

(57) **Abstract**

A cosmetic hair preparation comprising (A) one or multiple organopolysiloxanes having amino and polycaprolactone groups, represented by the general formula
(1) or (2) is provided. 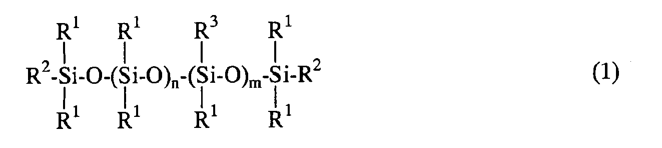 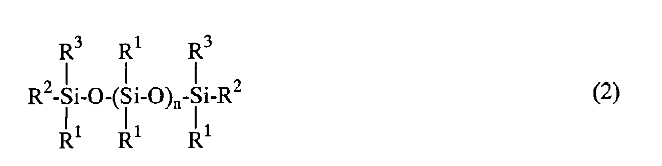 In formulae (1) and (2), R¹ is independently an unsubstituted monovalent hydrocarbon group of 1 to 20 carbon atoms, R² is independently R¹ or OX wherein X is hydrogen or R¹, R³ is an amino-containing organic group of formula (i):

-R⁴(NR⁵CH₂CH₂)ₐNR⁵₂ (i)

wherein R⁴ is a divalent hydrocarbon group of 1 to 6 carbon atoms, R⁵ is independently hydrogen or a polycaprolactone-containing organic group of formula (ii):

- (CO-C₅H₁₀O)_{b}-R⁶ (ii)

wherein R⁶ is hydrogen or a monovalent hydrocarbon group of 1 to 6 carbon atoms, b is an integer of 1 to 50, at least one of all R⁵ is the polycaprolactone-containing organic group, a is an integer of 0 to 3, n is an integer of 10 to 500, and m is an integer of 1 to 15.

## Description

### BACKGROUND

This invention relates to a cosmetic hair preparation comprising an organopolysiloxane having amino and polycaprolactone groups in a molecule. More particularly, it relates to a cosmetic hair preparation which can improve conditioning effects including setting, smoothness, ease of finger-combing and emollient effects on hair after drying, and improved usage due to the incorporation of such a specific organopolysiloxane and which can impart softness, slick and emollient effects due to addition of a hair-holding polymer as a setting agent, so that the preparation may possess better setting retention, be effective in inhibiting flaking of a coating of the hair-holding polymer, and have good stability over time.

Among cosmetic hair preparations including shampoos, rinses and treatments, the recent enhanced concern about hair care creates an increasing demand for those cosmetic hair preparations which are improved in conditioning effects including softness, smoothness, and emollient effects on hair after application, and have an appropriate setting ability.

In the prior art, cationic polymers such as cationic cellulose and silicone compounds are often used as the conditioning agent in shampoos. The cationic polymers can be readily washed away, but are highly adsorptive and accumulative and have a drawback that they cause greasy and bristly feels on use over a long period of time. The silicone compounds are improved in silkiness and smoothness, but not fully satisfactory in usage because of improper setting ability, short emollient effect, and development of hardness and coarseness. Several proposals were made for the enhancement of setting ability, including a combination of a specific polyamide-modified silicone with trimethylsiloxysilicic acid (JP-A 2006-199683), a combination of an amine oxide-containing resin with sphingolipid (JP-A 2005-239556), and a derivative of polysiloxane chain-containing ampholytic urethane resin (JP-A 2003-183138). They are not fully satisfactory in setting ability after drying.

Various hair-holding polymers are used for the purpose of imparting setting ability. Although these hair-holding polymers have a satisfactory setting force, they give rise to drawbacks when used in more amounts in order to gain a greater setting force, including detrimental effects on the tactile feel of hair as by rendering the hair more coarse and obstructive to finger-combing after drying, a flaking problem that a coating of the holding polymer spalls as white flakes on surfaces of hairs, and greasiness on drying.

To overcome these drawbacks, a combination of an anionic polymer with a specific polyether-modified silicone (JP-A 6-100418) and a combination of a branched fatty acid ester with a cationic surfactant (JP-B 6-96504) were proposed. They are not fully satisfactory as a cosmetic hair preparation for improving the tactile feel after setting.

A general aim of the present proposals, which have been developed in view of the mentioned background, is to provide a cosmetic hair preparation which has good or improved conditioning effects including setting, smoothness, ease of finger-combing and emollient effects on hair after drying, and improved usage, and which desirably imparts softness, slick and emollient effects when a setting agent is added, so that the preparation may possess better setting retention, be effective in inhibiting flaking of a coating of the setting agent, and have good stability over time.

The inventor has found that a cosmetic hair preparation comprising (A) an organopolysiloxane configured to have amino and polycaprolactone groups in a molecule and to exhibit specific properties has improved conditioning effects including appropriate setting, softness, smoothness, and emollient effects on hair.

The inventor has also found that when the organopolysiloxane (A) is combined with (B) a cationic surfactant, or with (C) an anionic surfactant and/or an ampholytic surfactant, there is obtained a cosmetic hair preparation which exhibits light spread, neat feel on use, and improved conditioning effects including setting, ease of finger-combing, softness, smoothness, and emollient effects on hair after application, and which is non-accumulative and pleasant to use and has good stability over time. It has also been found that when (D) a hair-holding polymer is blended as a setting agent in these cosmetic hair preparations, the resulting preparations are effective in imparting softness, slick and emollient effects and natural luster, and also in inhibiting flaking of a coating of the hair-holding polymer while possessing better setting retention, and have good stability over time.

Accordingly, the invention provides a cosmetic hair preparation comprising (A) one or multiple organopolysiloxanes having amino and polycaprolactone groups, represented by the general formula (1) or (2).

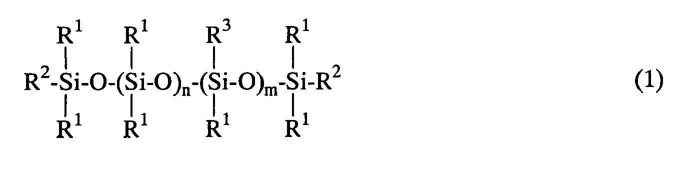

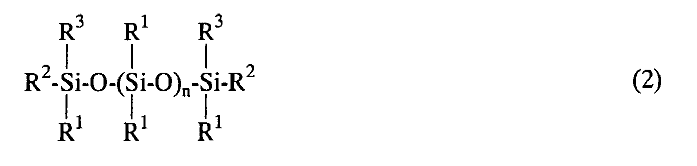

In formulae (1) and (2), R¹ is independently an unsubstituted monovalent hydrocarbon group of 1 to 20 carbon atoms,
R² is independently R¹ or OX wherein X is hydrogen or R¹,
R³ is an amino-containing organic group of formula (i):

-R⁴(NR⁵CH₂CH₂)ₐNR⁵₂ (i)

wherein R₄ is a divalent hydrocarbon group of 1 to 6 carbon atoms, R₅ is independently hydrogen or a polycaprolactone-containing organic group of formula (ii):

-(CO-C₅H₁₀O)_{b}-R⁶ (ii)

wherein R⁶ is hydrogen or a monovalent hydrocarbon group of 1 to 6 carbon atoms, b is an integer of 1 to 50, at least one of all R⁵ is the polycaprolactone-containing organic group, and a is an integer of 0 to 3,
n is an integer of 10 to 500, and m is an integer of 1 to 15.

In a preferred embodiment, the cosmetic hair preparation comprises 0.01 to 20% by weight of component (A). In formulae (1) and (2), R² is preferably R¹, OCH, or OC₂H₅; a is preferably 0 or 1; R⁶ is preferably hydrogen; and b is preferably 1 to 20. Further, in formulae (1) and (2), the polycaprolactone-containing organic group of formula -(CO-C₅H₁₀O)_{b}-R⁶ preferably accounts for at least 50 mol%, and more preferably at least 70 mol% of all R⁵.

The cosmetic hair preparation may further comprise one or more components selected from (B) a cationic surfactant, (C) an anionic surfactant and/or an ampholytic surfactant, and (D) a hair-holding polymer.

The cosmetic hair preparation having the organopolysiloxane compounded therein according to the invention spreads lightly on use, is pleasant and neat to use, and has improved conditioning effects including setting, ease of finger-combing, softness, smoothness, and emollient effects on hair after application, and is non-accumulative and has improved usage and good stability over time.

When a hair-holding polymer is further blended as the setting agent, the resulting cosmetic hair preparation is very effective in imparting softness, slick and emollient effects and natural luster, and also in inhibiting flaking of a coating of hair-holding polymer while possessing better setting retention, and has good stability over time.

The compositions themselves, methods of preparing them by combining their ingredients and their use on hair are aspects of the present proposals.

### FURTHER EXPLANATIONS; OPTIONS AND PREFERENCES

The invention provides a cosmetic hair preparation comprising (A) only one or multiple organopolysiloxanes having amino and polycaprolactone groups, represented by the general formula (1) or (2), preferably in an amount of 0.01 to 20% by weight.

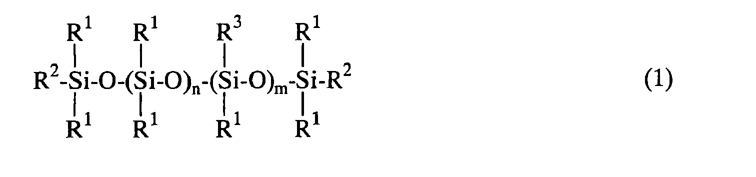

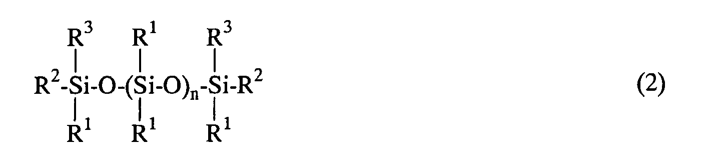

In formulae (1) and (2), R¹ is independently an unsubstituted monovalent hydrocarbon group of 1 to 20 carbon atoms,
R² is independently R¹ or OX wherein X is hydrogen or R¹,
R³ is an amino-containing organic group of formula (i):

-R⁴(NR⁵CH₂CH₂)ₐNR⁵₂ (i)

wherein R⁴ is a divalent hydrocarbon group of 1 to 6 carbon atoms, R⁵ is independently hydrogen or a polycaprolactone-containing organic group of formula (ii):

-(CO-C₅H₁₀O)_{b}-R⁶ (ii)

wherein R⁶ is hydrogen or a monovalent hydrocarbon group of 1 to 6 carbon atoms, b is an integer of 1 to 50, at least one of all R⁵ is the polycaprolactone-containing organic group, and a is an integer of 0 to 3,
n is an integer of 10 to 500, and m is an integer of 1 to 15.

Component (A) used herein consists of only one or multiple organopolysiloxanes having amino and polycaprolactone groups, represented by the general formula (1) or (2).

In formulae (1) and (2), R¹ is an unsubstituted monovalent hydrocarbon group of 1 to 20 carbon atoms. Illustrative examples of R¹ include alkyl groups such as methyl, ethyl, propyl, butyl, pentyl, hexyl, heptyl, octyl, nonyl, decyl, dodecyl, tetradecyl, hexadecyl, octadecyl, and eicosyl; cycloalkyl groups such as cyclopentyl and cyclohexyl; aryl groups such as phenyl and tolyl; aralkyl groups such as benzyl; and alkenyl groups such as vinyl and allyl. Of these groups, methyl is most preferred from the industrial aspect.

R² is R¹ or OX wherein X is hydrogen or a monovalent hydrocarbon group like R¹. Examples of the monovalent hydrocarbon group represented by X include alkyl groups such as methyl, ethyl, propyl, butyl, pentyl, hexyl, heptyl, and octyl, with methyl and ethyl being preferred. More preferably R² is R¹, OCH₃ or OC₂H₅. Preferences for R¹ may apply.

In formulae (1) and (2), R³ is an amino group of formula (i).

-R⁴(NR⁵CH₂CH₂)ₐNR⁵₂ (i)

Examples of R⁴ include alkylene groups such as methylene, dimethylene, trimethylene, and tetramethylene, with trimethylene being preferred. The subscript "a" is an integer of 0 to 3, and most preferably 0 or 1.
R⁵ is independently hydrogen or a polycaprolactone-containing organic group of formula (ii), and at least one of all R⁵ is the polycaprolactone-containing organic group.

-(CO-C₅H₁₀O)_{b}-R⁶ (ii)

In the polycaprolactone-containing organic group of formula (ii) represented by R⁵, b is an integer of 1 to 50 because setting property is insufficient if b is less than 1 and softness is insufficient if b is more than 50. Preferably b is an integer of 1 to 20.

R⁶ is hydrogen or a monovalent hydrocarbon group of 1 to 6 carbon atoms. Illustrative examples of R⁶ include hydrogen or alkyl groups such as methyl, ethyl, propyl, and butyl, with hydrogen being preferred.

To gain better effects including setting, softness, smoothness, and emollient effects on hair, the polycaprolactone-containing organic group preferably accounts for at least 50 mol%, and more preferably at least 70 mol% of all R⁵.

In formulae (1) and (2), n should be an integer of 10 to 500, and preferably an integer of 50 to 300, because endowment of hair with softness, slick and setting properties is insufficient if n is less than 10, and hair is rendered greasy and coarse if n is more than 500.

In formula (1), m is an integer of 1 to 15, and preferably an integer of 1 to 10. Endowment of hair with softness and setting properties is insufficient if m is 0, and hair is rendered greasy and coarse if m is more than 15.

The organopolysiloxanes of formulae (1) and (2) used as component (A) in the cosmetic hair preparation of the invention may be obtainable by ring-opening addition polymerization of organopolysiloxanes of the following general formulae (3) and (4) with ε-caprolactone of the following formula (5).

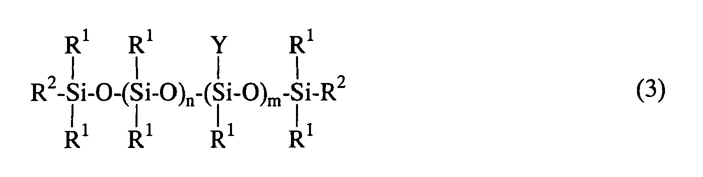

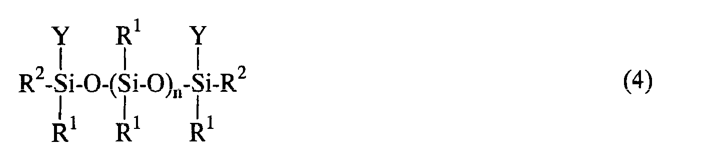

Herein R¹ and R² are as defined above, Y is an amino group of formula (iii):

-R⁴(NHCH₂CH₂)ₐNH₂ (iii)

wherein R⁴ and a are as defined above, and n and m are as defined above.

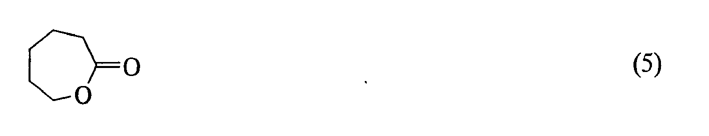

Examples of the organopolysiloxanes of the general formulae (3) and (4) include the following, but are not limited thereto. 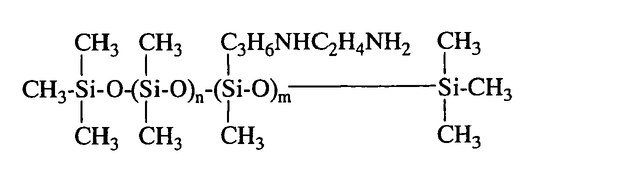 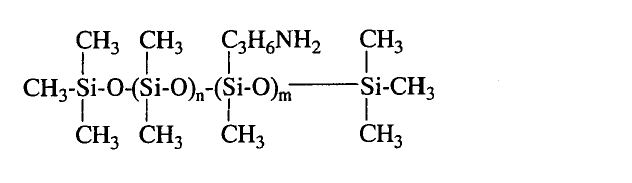 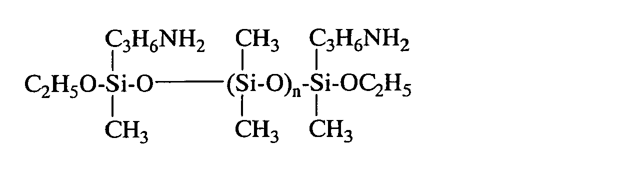 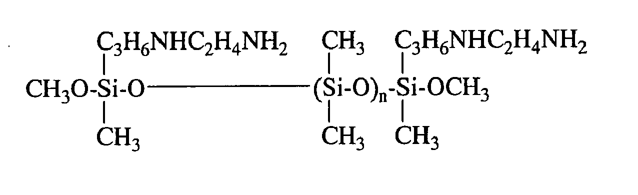 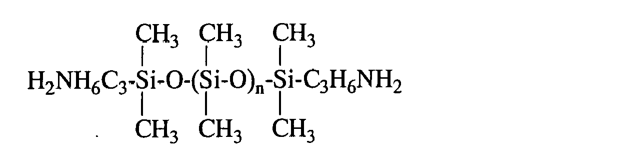 Herein n and m are as defined above.

As mentioned above, it is preferred for improving setting, softness, smoothness, and emollient effects on hair that the content of polycaprolactone-containing organic group of formula (ii) be at least 50 mol%, and more preferably at least 70 mol% of all R⁵ in general formula (1) or (2). It is then preferred that reaction be performed under such conditions that ε-caprolactone of formula (5) be at least 50 mol%, and more preferably at least 70 mol% based on entire NH groups in the organopolysiloxane of formula (3) or (4).

More specifically, the proportion of organopolysiloxane of formula (3) or (4) to ε-caprolactone of formula (5) used in reaction is preferably such that 1 to 50 moles of ε-caprolactone is reacted per mole of NH groups in the organopolysiloxane, and more preferably 1 to 20 moles of ε-caprolactone is reacted. Too small an amount of ε-caprolactone may lead to insufficient setting property whereas too large an amount of ε-caprolactone may lead to insufficient softness.

The reaction of NH-containing organopolysiloxane of formula (3) or (4) with ε-caprolactone of formula (5) may be performed at 110 to 140°C for 2 to 5 hours in a solvent such as toluene or xylene, using a catalyst. The preferred catalysts are titanium base catalysts such as tetraalkoxytitanium.

In the inventive cosmetic hair preparation, the organopolysiloxane as component (A) is preferably blended in an amount of 0.01 to 20% by weight, and more preferably 0.05 to 10% by weight, for the development of the desired effects and usage. If the blending amount is less than 0.01% by weight, sometimes the desired effects may not be obtained. An amount in excess of 20% by weight may exacerbate usage.

Component (A) may consist of one organopolysiloxane of formula (1) or (2) or a combination of any two or more organopolysiloxanes of formulae (1) and (2) wherein a mixing ratio is not particularly limited.

In embodiments of the invention wherein the organopolysiloxane (A) is combined with (B) a cationic surfactant or with (C) an anionic surfactant and/or an ampholytic surfactant, there may be obtained cosmetic hair preparations which exhibit light spread, neat feel on use, and improved conditioning effects including gathering, ease of finger-combing, softness, smoothness, emollient, and appropriate setting effects on hair after application, and are non-accumulative and convenient to use.

If desired, in a further embodiment wherein (D) a hair-holding polymer is incorporated to formulate a hair conditioner or dresser, there may be obtained a hair conditioner which does not render hair greasy or coarse, spreads lightly, has neat feel on use, silky and smooth tactile feel, and emollient effects, inhibits greasiness and flaking of a coating of the holding polymer on drying, and has good setting retention.

The cationic surfactant used herein as component (B) may be any of such surfactants which are used in ordinary cosmetic preparations. Examples include alkyltrimethylammonium chloride, stearyltrimethylammonium chloride, lauryltrimethylammonium chloride, cetyltrimethylammonium chloride, beef tallow alkyltrimethylammonium chloride, behenyltrimethylammonium chloride, stearyltrimethylammonium bromide, behenyltrimethylammonium bromide, distearyldimethylammonium chloride, dicocoyldimethylammonium chloride, dioctyldimethylammonium chloride, polyoxyethylene oleylmethylammonium (2EO) chloride, benzalkonium chloride, stearyldimethylbenzylammonium chloride, lanolin-derived quaternary ammonium salt, stearic acid diethylaminoethylamide, stearic acid dimethylaminopropylamide, and behenic acid amidopropyldimethylhydroxypropylammonium chloride.

In the inventive cosmetic hair preparation, component (B) is preferably blended in a proportion of 0.01 to 20% by weight, and more preferably 0.05 to 10% by weight, in order to impart pleasant feels such as smooth and silky feels to hair. If the blending proportion is less than 0.01% by weight, the conditioning effects on hair may be insufficient. A proportion in excess of 20% by weight may lead to unpleasant feels such as oily feel.

The anionic surfactant and/or ampholytic surfactant used herein as component (C) may be any of such surfactants which are used in ordinary cosmetic preparations. Examples of the anionic surfactant include saturated or unsaturated fatty acid soaps, polyoxyethylene alkylsulfate salts, α-acylsulfonic acid salts, alkylsulfonic acid salts, alkylallylsulfonic acid salts, α-olefinsulfonic acid salts, alkylbenzenesulfonic acid salts, alkylnaphthalenesulfonic acid salts, alkanesulfonic acid salts, alkyl or alkenyl-sulfuric acid salts, alkylamidosulfuric acid salts, alkyl or alkenyl-phosphoric acid salts, alkylamidophosphoric acid salts, alkyloylalkyltaurin salts, N-acylamino acid salts, sulfosuccinic acid salts, alkyl ether carboxylic acid salts, amido ether carboxylic acid salts, and α-sulfofatty acid ester salts.

Examples of the ampholytic surfactant include carboxybetaine, amidobetaine, sulfobetaine, hydroxysulfobetaine, amidosulfobetaine, phosphobetaine, aminocarboxylic acid salt, imidazoline derivative, and amidoamine compounds. It is noted that when both an anionic surfactant and an ampholytic surfactant are used in combination as component (C), a blending ratio of them is arbitrary.

In the inventive cosmetic hair preparation, component (C) is preferably blended in a proportion of 0.01 to 20% by weight, and more preferably 0.05 to 10% by weight, in order to impart pleasant feels such as smooth and silky feels to hair. If the blending proportion is less than 0.01% by weight, the conditioning effects on hair may be insufficient. A proportion in excess of 20% by weight may lead to unpleasant feels such as oily feel.

Examples of the polymer suitable as component (D) are known for this use and include ampholytic, anionic, cationic and nonionic polymers. Polyvinylpyrrolidone polymers include polyvinylpyrrolidone, vinylpyrrolidone/vinyl acetate copolymers, vinylpyrrolidone/vinyl acetate/vinyl propionate ternary copolymers, vinylpyrrolidone/alkylamino(meth)acrylate (quaternized) copolymers, vinylpyrrolidone/alkyl (meth)acrylate/(meth)acrylic acid copolymers, vinylpyrrolidone/alkylamino(meth)acrylate/vinyl caprolactam copolymers, and vinylpyrrolidone/methylvinylimidazolium chloride; acidic vinyl ether polymers include methyl vinyl ether/maleic anhydride alkyl half-ester copolymers; acidic vinyl acetate polymers include vinyl acetate/crotonic acid copolymers and vinyl acetate/crotonic acid/vinyl neodecanoate copolymers; acidic acrylic polymers include (meth)acrylic acid/alkyl (meth)acrylate copolymers and (meth)acrylic acid/alkyl (meth)acrylate/alkyl acrylamide copolymers; ampholytic acrylic polymers include N-methacryloylethyl-N,N-dimethylammonium α-N-methylcarboxybetaine/alkyl (meth)acrylate copolymers and hydroxypropyl (meth)acrylate/butylaminoethyl methacrylate/acrylic acid octylamide copolymers. Also naturally occurring polymers including cellulose or derivatives thereof, keratin and collagen or derivatives thereof may preferably be used.

In embodiments wherein ampholytic or anionic hair-holding polymers are used, some or all of functional groups therein may be neutralized with organic amines such as 2-amino-2-methyl-1-propanol and triethanol amine and alkaline agents such as potassium hydroxide, if necessary. The hair-holding polymer (D) used herein may also be a single polymer or a mixture of two or more polymers.

In the inventive cosmetic hair preparation, the hair-holding polymer used as component (D) is preferably blended in a proportion of 0.01 to 20% by weight, and more preferably 0.1 to 10% by weight, for the development of the desired effects and usage. If the blending proportion is less than 0.01% by weight, sometimes the desired effects may not be obtained. A proportion in excess of 20% by weight may exacerbate usage.

In addition to the foregoing components, other components which are commonly used in cosmetic hair preparations, such as viscosity modifiers, film formers, hair quality modifiers, pH modifiers, cleaning agents, emulsifying agents, emulsifying aids, and propellants may be formulated in the cosmetic hair preparation of the invention for a particular purpose as long as the effects of the invention are not compromised.

Suitable viscosity modifiers which can be used herein include water-soluble polymers such as methyl cellulose, carboxymethyl cellulose, hydroxyethyl cellulose, and xanthane gum, and nonionic surfactants such as fatty acid alkylol amides. Suitable film formers which can be used herein include cationic polymers such as cationic cellulose, cationic starch, cationic guar gum, quaternized vinylpyrrolidone-N,N-dimethylaminoethyl methacrylic acid copolymers, and diallyl quaternary ammonium salt polymers; nonionic polymers such as polyvinylpyrrolidone, vinylpyrrolidone-vinyl acetate copolymers, and polyvinyl alcohol; anionic polymers such as methyl vinyl ether-maleic acid half-ester copolymers and acrylic resin in alkanolamine; and ampholytic copolymers such as N-methacryloyloxyethylene-N,N-dimethylammonium α-N-methylcarboxybetaine-alkyl methacrylate copolymers.

Suitable hair quality modifiers which can be used herein include silicone derivatives such as low-viscosity silicones, highly polymerized silicones, cyclic silicones, polyether-modified silicones, amino-modified silicones, and cationic silicones; suitable pH modifiers include acids such as citric acid and lactic acid or salts thereof; suitable emulsifying agents include nonionic surfactants such as polyoxyalkylene added surfactants; suitable emulsifying aids include higher alcohols and glycerol fatty acid esters; and suitable propellants include liquefied petroleum gas, nitrogen gas, carbonic acid gas, and dimethyl ether.

Additionally, other components which are commonly blended in cosmetic preparations may be blended, for example, oily components such as higher fatty acids, straight or branched chain esters, hydrocarbons, oil and fats; aqueous components such as polyhydric alcohols and lower alcohols; perfume, preservatives, UV-absorbers, antioxidants, antibacterial or bactericidal agents, humectants, salts, chelates, refreshing agents, anti-inflammatory agents, skin-improving components (e.g., whitening agents, cell activators, anti-skin-roughening agents, blood flow promoting agents, skin astringents, and anti-seborrheic agents), vitamins, amino acids, nucleic acids, hormones, inclusion compounds, and the like.

The cosmetic hair preparation of the invention may be embodied in any of various forms including liquid, emulsion, cream, solid, paste, gel, mousse, and mist forms by combining it with other components or using a certain mechanism of container. That is, its dosage form is not critical. The method for manufacturing the cosmetic hair preparation of the invention is not particularly limited, and it may be manufactured by any well-known techniques.

### EXAMPLE

Examples are given below for illustrating the invention in detail, but the invention is not limited thereto. In Examples, the viscosity is as measured at 25°C by a rotational viscometer, the amine equivalent is as measured by the neutralization titration method; and all percents in Tables are by weight.

### Preparation Example 1

A four-neck separable flask of 1000-ml volume equipped with a thermometer, stirrer, reflux condenser, and nitrogen gas inlet tube was charged with 400 g of an amino-containing organopolysiloxane of the following formula (A) (viscosity 1,800 mPa-s, amine equivalent 3,800 g/mol), 120 g (corresponding to 5-fold moles relative to entire NH groups in the amino-containing organopolysiloxane) of ε-caprolactone of the following formula (5) (molecular weight 114), 300 g of toluene, and 0.2 g of a titanium base catalyst (tetrabutoxytitanium, TBT100 by Nippon Soda Co., Ltd., same hereinafter). The flask was purged with nitrogen gas and closed, after which reaction was allowed to run at 110°C for 5 hours. After the completion of reaction, a low-boiling fraction was removed under a vacuum of 10 mmHg at 80°C for 1 hour, yielding 495 g of an oily matter having a pale yellow clear appearance, a viscosity of 230,000 mPa-s (25°C), and an amine equivalent unmeasurable. The structure of the oily matter was examined by nuclear magnetic resonance spectroscopy (¹H-NMR), gel permeation chromatography (GPC), and Fourier transform infrared spectroscopy (FTIR). It was identified to be a polycaprolactone/amino-containing organopolysiloxane in which about 5 moles of caprolactone was added to entire amino groups in the amino-containing organopolysiloxane, as represented by the following formula (A-1).

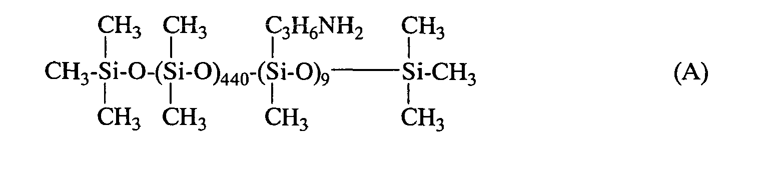

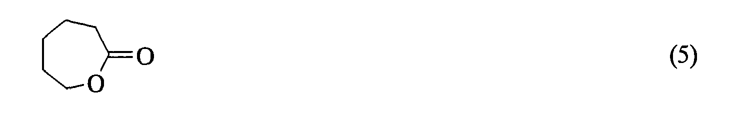

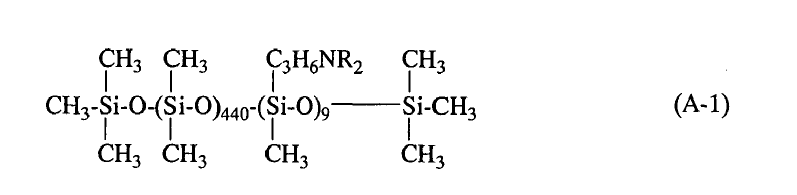

R : -(COC₅H₁₀O)₅-H

### Preparation Example 2

A four-neck separable flask of 1000-ml volume equipped with a thermometer, stirrer, reflux condenser, and nitrogen gas inlet tube was charged with 200 g of an amino-containing organopolysiloxane of the following formula (B) (viscosity 1,300 mPa-s, amine equivalent 1,700 g/mol), 100 g (corresponding to 5-fold moles relative to entire NH groups in the amino-containing organopolysiloxane) of ε-caprolactone of formula (5) (molecular weight 114), 300 g of toluene, and 0.2 g of the titanium base catalyst. The flask was purged with nitrogen gas and closed, after which reaction was allowed to run at 110°C for 5 hours. After the completion of reaction, a low-boiling fraction was removed under a vacuum of 10 mmHg at 80°C for 1 hour, yielding 280 g of an oily matter having a pale yellow clear appearance, a viscosity of 55,000 mPa-s (25°C), and an amine equivalent of 5,400 g/mol. The structure of the oily matter was examined by nuclear magnetic resonance spectroscopy (¹H-NMR), gel permeation chromatography (GPC), and Fourier transform infrared spectroscopy (FTIR). It was identified to be a polycaprolactone/amino-containing organopolysiloxane in which about 5 moles of caprolactone was added to about 72% of amino groups in the amino-containing organopolysiloxane, as represented by the following formula (B-1).

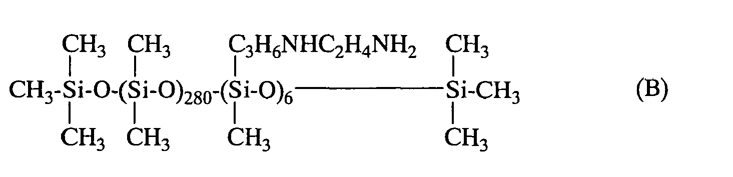

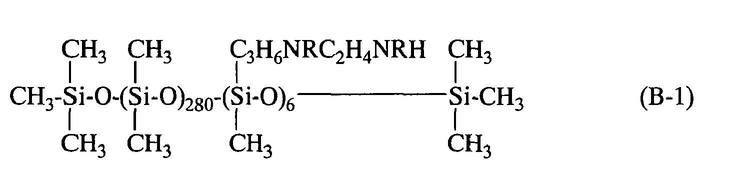

R : -(COC₅H₁₀O)₅-H

### Preparation Example 3

A four-neck separable flask of 1000-ml volume equipped with a thermometer, stirrer, reflux condenser, and nitrogen gas inlet tube was charged with 200 g of an amino-containing organopolysiloxane of the following formula (C) (viscosity 110 mPa-s, amine equivalent 1,550 g/mol), 120 g (corresponding to 4-fold moles relative to entire NH groups in the amino-containing organopolysiloxane) of ε-caprolactone of formula (5) (molecular weight 114), 300 g of toluene, and 0.2 g of the titanium base catalyst. The flask was purged with nitrogen gas and closed, after which reaction was allowed to run at 110°C for 5 hours. After the completion of reaction, a low-boiling fraction was removed under a vacuum of 10 mmHg at 80°C for 1 hour, yielding 510 g of an oily matter having a pale yellow clear appearance, a viscosity of 5,800 mPa-s (25°C), and an amine equivalent unmeasurable.
The structure of the oily matter was examined by nuclear magnetic resonance spectroscopy (¹H-NMR), gel permeation chromatography (GPC), and Fourier transform infrared spectroscopy (FTIR). It was identified to be a polycaprolactone/amino-containing organopolysiloxane in which about 4 moles of caprolactone was added to entire amino groups in the amino-containing organopolysiloxane, as represented by the following formula (C-1).

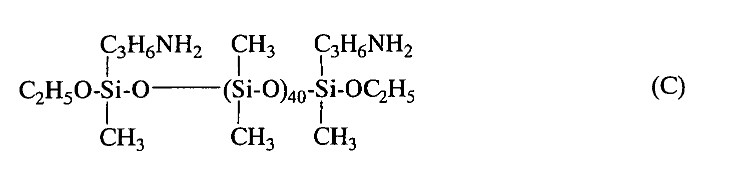

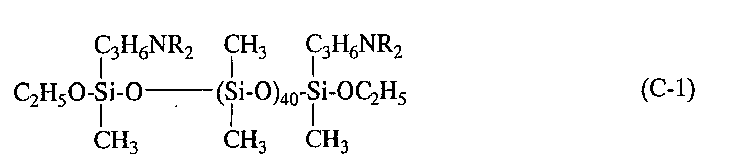

### R : -(COC₅H₁₀O)₄-H

### Preparation Example 4

A four-neck separable flask of 1000-ml volume equipped with a thermometer, stirrer, reflux condenser, and nitrogen gas inlet tube was charged with 200 g of an amino-containing organopolysiloxane of the following formula (D) (viscosity 25 mPa-s, amine equivalent 800 g/mol), 230 g (corresponding to 4-fold moles relative to entire NH groups in the amino-containing organopolysiloxane) of ε-caprolactone of formula (5) (molecular weight 114), 300 g of toluene, and 0.2 g of the titanium base catalyst. The flask was purged with nitrogen gas and closed, after which reaction was allowed to run at 110°C for 5 hours. After the completion of reaction, a low-boiling fraction was removed under a vacuum of 10 mmHg at 80°C for 1 hour, yielding 510 g of an oily matter having a pale yellow clear appearance, a viscosity of 2,800 mPa-s (25°C), and an amine equivalent unmeasurable. The structure of the oily matter was examined by nuclear magnetic resonance spectroscopy (¹H-NMR), gel permeation chromatography (GPC), and Fourier transform infrared spectroscopy (FTIR). It was identified to be a polycaprolactone/amino-containing organopolysiloxane in which about 4 moles of caprolactone was added to entire amino groups in the amino-containing organopolysiloxane, as represented by the following formula (D-1).

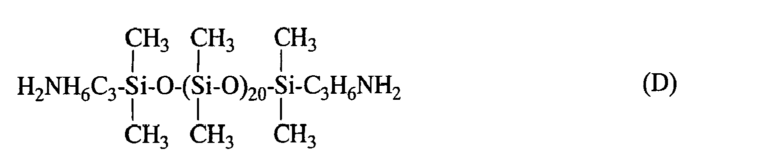

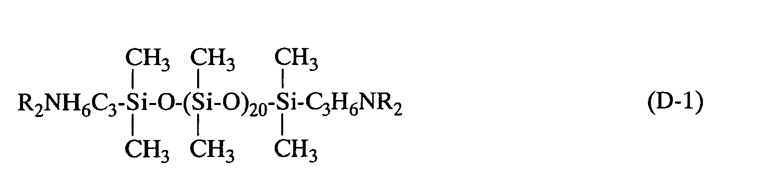

### R : -(COC₅H₁₀O)₄-H

### Examples 1 and 2 and Comparative Examples 1 to 3

### Hair conditioner

Hair conditioners of the formulation shown in Table 1 were prepared and examined for feel on use and usage by the following test procedure. The results are also shown in Table 1.

**Table 1**

| Component (%) | Example | | Comparative Example | | |
|---|---|---|---|---|---|
| | 1 | 2 | 1 | 2 | 3 |
| 1. Alkyltrimethylammonium chloride | 2 | - | 2 | 2 | - |
| 2. Distearyldimethylammonium chloride | - | 2 | - | - | 2 |
| 3. Cetanol | 5 | 3 | 5 | 5 | 3 |
| 4. Behenyl alcohol | - | 3 | - | - | 3 |
| 5. Organopolysiloxane (A-1) (Preparation Example 1) | 7 | 3 | - | - | - |
| 6. Methylpolysiloxane (6 mm²/s @25°C) | - | 5 | 5 | 10 | 8 |
| 7. Highly polymerized silicone emulsion*¹ | - | - | - | 5 | - |
| 8. Amino-modified silicone emulsion*² | - | - | 5 | - | - |
| 9. Propylene glycol | 10 | 10 | 10 | 10 | 10 |
| 10. Preservative | appropriate | appropriate | appropriate | appropriate | appropriate |
| 11. Perfume | appropriate | appropriate | appropriate | appropriate | appropriate |
| 12. Purified water | balance | balance | balance | balance | balance |
| Test items | | | | | |
| 1. Spread on application | ⊚ | ⊚ | △ | × | × |
| 2. Finger-combing on rinsing | ⊚ | ⊚ | × | × | × |
| 3. Setting after drying | ⊚ | ⊚ | × | × | × |
| 4. Smoothness | ⊚ | ⊚ | △ | △ | × |
| 5. Silky feel | ⊚ | ⊚ | △ | × | × |
| 6. Overall rating | ⊚ | ⊚ | × | × | × |

| | | | | | |
|---|---|---|---|---|---|
| *1 Highly polymerized silicone emulsion, KM-903 (trade name, Shin-Etsu Chemical Co., Ltd.) *2 Amino-modified silicone emulsion, KM-907 (trade name, Shin-Etsu Chemical Co., Ltd.) | | | | | |

### Process for preparing hair conditioner

Step A: Mix components 1, 2, 9, 10 and 12 and dissolve homogeneously while heating.
Step B: Mix components 3 to 8 and dissolve homogeneously while heating.
Step C: Add the mixture of step B to the mixture of step A, emulsify, cool, and add component 11, yielding a hair conditioner.

### Test procedure

A panel of ten female specialists conducts a use test by washing hair with a commercially available shampoo, applying the hair conditioner, evaluating the spread upon application and ease of finger combing upon rinsing according to the following criterion, then drying the hair, evaluating the setting, smoothness and silky feel of the hair after drying according to the following criterion, and rating by an average point.

### Evaluating criterion

Point 5: excellent
Point 4: good
Point 3: ordinary
Point 2: somewhat poor
Point 1: poor

### Rating

⊚: 4.5 ≤ average point
○: 3.5 ≤ average point < 4.5
△: 2.5 ≤ average point < 3.5
×: average point < 2.5

It is evident from the data of Table 1 that as compared with the hair conditioners of Comparative Examples 1 to 3, the hair conditioners of Examples 1 and 2 having blended therein the organopolysiloxane within the scope of the invention show significantly improved effects with respect to spread upon application, finger combing upon rinsing, setting, smoothness and silky feel of hair after drying, indicating an overall excellent profile of properties.

### Examples 3 to 5 and Comparative Examples 4 to 6

### Hair cream

Hair creams of the formulation shown in Table 2 were prepared and examined for feel on use and usage. The results are also shown in Table 2.

**Table 2**

| Component (%) | Example | | | Comparative Example | | |
|---|---|---|---|---|---|---|
| | 3 | 4 | 5 | 4 | 5 | 6 |
| 1. Decamethylcyclopentasiloxane | 7 | 7 | 7 | 7 | 7 | 7 |
| 2. Organopolysiloxane (B-1) (Preparation Example 2) | 1 | 5 | - | - | - | - |
| 3. Organopolysiloxane (C-1) (Preparation Example 3) | - | - | 5 | - | - | - |
| 4. Isopropyl myristate | - | - | - | 5 | - | 3 |
| 5. Methylpolysiloxane (10 mm²/s @25°C) | 5 | - | - | - | 5 | 3 |
| 6. Stearyltrimethylammonium chloride | 1.5 | 1.5 | 1.5 | 1.5 | 1.5 | 1.5 |
| 7. Cetanol | 2 | 2 | 2 | 2 | 2 | 2 |
| 8. Propylene glycol | 5 | 5 | 5 | 5 | 5 | 5 |
| 9. Glycerol | 3 | 3 | 3 | 3 | 3 | 3 |
| 10. Hydroxyethyl cellulose | 0.2 | 0.2 | 0.2 | 0.2 | 0.2 | 0.2 |
| 11. Preservative | appropriate | appropriate | appropriate | appropriate | appropriate | appropriate |
| 12. Perfume | appropriate | appropriate | appropriate | appropriate | appropriate | appropriate |
| 13. Purified water | balance | balance | balance | balance | balance | balance |
| Test items | | | | | | |
| 1. Spread on application | ⊚ | ⊚ | ⊚ | × | × | × |
| 2. Softness after use | ⊚ | ⊚ | ⊚ | △ | × | × |
| 4. Smoothness | ⊚ | ⊚ | ⊚ | × | △ | × |
| 4. Setting | ⊚ | ⊚ | ⊚ | × | × | × |
| 5. Silky feel | ⊚ | ⊚ | ⊚ | × | × | × |
| 6. Overall rating | ⊚ | ⊚ | ⊚ | × | × | × |

### Process for preparing hair cream

Step A: Dissolve components 1 to 5 and 7 homogeneously while heating.
Step B: Mix components 6, 8 to 11 and 13 and dissolve homogeneously while heating.
Step C: Add the mixture of step A to the mixture of step B, emulsify, cool, and add component 12, yielding a hair cream.

### Rating

With respect to spread upon application, softness, smoothness, setting and silky feel of hair after use, evaluation was made according to the criterion described above. An average point gives a rating.

It is evident from the data of Table 2 that as compared with the hair creams of Comparative Examples 4 to 6, the hair creams of Examples 3 to 5 having blended therein the organopolysiloxane within the scope of the invention show significantly improved effects with respect to spread upon application, softness, smoothness, setting and silky feel of hair after use, indicating an overall excellent profile of properties.

### Examples 6 to 8 and Comparative Examples 7 to 9

### Styling mousse

Styling mousses of the formulation shown in Table 3 were prepared and examined for feel on use and usage. The results are also shown in Table 3.

**Table 3**

| Component (%) | Example | | | Comparative Example | | |
|---|---|---|---|---|---|---|
| | 6 | 7 | 8 | 7 | 8 | 9 |
| 1. Vinylpyrrolidone/ vinyl acetate copolymer*¹ | 5 | - | - | 5 | - | - |
| 2. Methyl vinyl ether/butyl maleate half-ester copolymer*² | - | 3 | - | - | 3 | - |
| 3. Vinylpyrrolidone/ dimethylaminoethyl methacrylate copolymer diethylsulfate*³ | - | - | 5 | - | - | 5 |
| 4. Organopolysiloxane (D-1) (Preparation Example 4) | 1 | 2 | 0.5 | - | - | - |
| 5. Polyether-modified silicone*⁴ | - | - | - | - | 1 | - |
| 6. Highly polymerized silicone emulsion*⁵ | - | - | - | 1 | - | - |
| 7. Ethanol | 10 | 10 | 10 | 10 | 10 | 10 |
| 8. Preservative | appropriate | appropriate | appropriate | appropriate | appropriate | appropriate |
| 9. Perfume | appropriate | appropriate | appropriate | appropriate | appropriate | appropriate |
| 10. Purified water | balance | balance | balance | balance | balance | balance |
| 11. Liquefied petroleum gas | 7 | 7 | 7 | 7 | 7 | 7 |
| Test items | | | | | | |
| 1. Setting force | ⊚ | ⊚ | ⊚ | △ | ○ | × |
| 2. Setting retention | ⊚ | ⊚ | ⊚ | △ | × | × |
| 3. Texture of hair | ⊚ | ⊚ | ⊚ | × | × | × |
| 4. Smoothness | ⊚ | ⊚ | ⊚ | ○ | × | △ |
| 5. Luster | ⊚ | ⊚ | ⊚ | × | × | × |
| 6. Non-flaking | ⊚ | ⊚ | ⊚ | × | × | △ |
| 7. Overall rating | ⊚ | ⊚ | ⊚ | × | × | × |

| | | | | | | |
|---|---|---|---|---|---|---|
| *1 Vinylpyrrolidone/vinyl acetate copolymer: PVP/VA E-735 (by GAF) *2 Methyl vinyl ether/butyl maleate half-ester copolymer: BEM-42S(N) (by Osaka Organic Chemical Industry Ltd.) *3 Vinylpyrrolidone/dimethylaminoethyl methacrylate copolymer diethyl sulfate solution: HC Polymer 3A (by Osaka Organic Chemical Industry Ltd.) *4 Polyether-modified silicone: KF-6017 (Shin-Etsu Chemical Co., Ltd.) *5 Highly polymerized silicone emulsion: KM-903 (Shin-Etsu Chemical Co., Ltd.) | | | | | | |

### Process for Preparing styling mousse

Step A: Mix components 1 to 10 homogeneously.
Step B: Fill an aerosol can with the mixture of step A together with component 11, yielding a styling mousse.

### Rating

A wig of human hair is treated with a commercial ordinary shampoo and rinse and then dried. Next, a 5-g sample of each of Examples 6 to 8 and Comparative Examples 7 to 9 is applied to the wig of human hair, which is dried and evaluated for setting force, setting retention, texture of hair including greasy and coarse feels, smoothness, and luster. The flaking state is determined by combing the wig 5 times, checking the absence of flakes and making evaluation according to the foregoing criterion. An average point gives a rating.

It is evident from the data of Table 3 that as compared with the styling mousses of Comparative Examples 7 to 9, the styling mousses of Examples 6 to 8 having blended therein the organopolysiloxane within the scope of the invention show significantly improved effects with respect to setting force, setting retention, texture of hair including greasy and coarse feels, smoothness, luster, and flaking state, indicating an overall excellent profile of properties.

### Note

In respect of numerical ranges disclosed in the present description it will of course be understood that in the normal way the technical criterion for the upper limit is different from the technical criterion for the lower limit, i.e. the upper and lower limits are intrinsically distinct proposals.

## Claims

1. A cosmetic hair preparation comprising (A) one or more organopolysiloxanes having amino and; polycaprolactone groups, represented by the general formula (1) or (2): 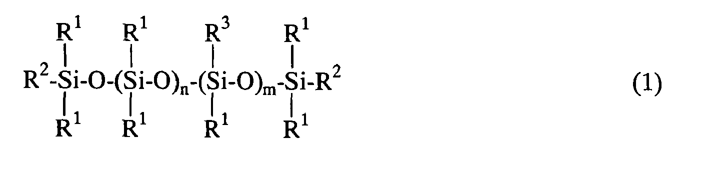 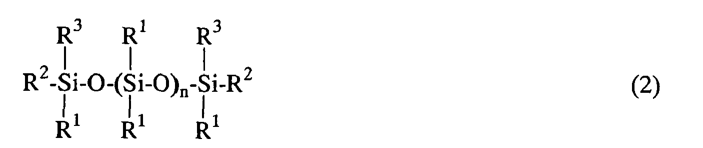 where groups R¹ are selected independently from unsubstituted monovalent hydrocarbon groups of 1 to 20 carbon atoms,
groups R² are selected independently from the above options for R¹ and OX, where X is hydrogen or group selected from the options for R¹;
R³ is an amino-containing organic group of formula (i):
-R⁴(NR⁵CH₂CH₂)ₐNR⁵₂ (i)
wherein R⁴ is a divalent hydrocarbon group of 1 to 6 carbon atoms, R⁵ is independently hydrogen or a polycaprolactone-containing organic group of formula (ii):
-(CO-C₅H₁₀O)_{b}-R⁶ (ii)
wherein R⁶ is hydrogen or a monovalent hydrocarbon group of 1 to 6 carbon atoms, b is an integer of 1 to 50, at least one of all R⁵ is the polycaprolactone-containing organic group, and a is an integer of 0 to 3,
n is an integer of 10 to 500, and m is an integer of 1 to 15.

2. The cosmetic hair preparation of claim 1, comprising 0.01 to 20% by weight of component (A).

3. The cosmetic hair preparation of claim 1 or 2 wherein in formulae (1) and (2), R² is R¹, OCH₃ or OC₂H₅.

4. The cosmetic hair preparation of claim 1, 2 or 3 wherein in formulae (1) and (2), a is 0 or 1.

5. The cosmetic hair preparation of any one of claims 1 to 4 wherein in formulae (1) and (2), the polycaprolactone-containing organic group of formula -(CO-C₅H₁₀O)_{b}-R⁶ accounts for at least 50 mol% of all R⁵.

6. The cosmetic hair preparation of any one of claims 1 to 4 wherein in formulae (1) and (2), the polycaprolactone-containing organic group of formula -(CO-C₅H₁₀O)_{b}-R⁶ accounts for at least 70 mol% of all R⁵.

7. The cosmetic hair preparation of any one of claims 1 to 6 wherein in formulae (1) and (2), R⁶ is hydrogen.

8. The cosmetic hair preparation of any one of claims 1 to 7 wherein in formulae (1) and (2), b is 1 to 20.

9. The cosmetic hair preparation of any one of claims 1 to 8, further comprising (B) a cationic surfactant.

10. The cosmetic hair preparation of any one of claims 1 to 9, further comprising (C) an anionic surfactant and/or an ampholytic surfactant.

11. The cosmetic hair preparation of any one of claims 1 to 10, further comprising (D) a hair-holding polymer.

12. A cosmetic hair treatment in which a preparation as defined in any one of claims 1 to 11 is applied to a person's hair.
